(19) Europäisches Patentamt
European Patent Office
Office européen des brevets

(11) EP 1 433 848 A1

(12) EUROPEAN PATENT APPLICATION
published in accordance with Art. 158(3) EPC

(43) Date of publication:
**30.06.2004 Bulletin 2004/27**

(21) Application number: **02762987.2**

(22) Date of filing: **03.09.2002**

(51) Int Cl.[7]: **C12N 15/12**, C12N 9/99, C12N 5/10, C12Q 1/68, C12Q 1/02, C07K 16/38

(86) International application number:
**PCT/JP2002/008948**

(87) International publication number:
**WO 2003/020933 (13.03.2003 Gazette 2003/11)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **03.09.2001 JP 2001266004**

(71) Applicant: **MOCHIDA PHARMACEUTICAL CO., LTD.**
**Shinjuku-ku Tokyo 160-8515 (JP)**

(72) Inventors:
- **SHINOZAKI, Mikihiko,**
**MOCHIDA PHARMACEUTICAL CO.**
**Shinjuku-ku, Tokyo 160-8515 (JP)**
- **HOSAKA, Yoshitaka,**
**MOCHIDA PHARMACEUTICAL CO.**
**Shinjuku-ku, Tokyo 160-8515 (JP)**
- **NANBA, Mutsuo,**
**MOCHIDA PHARMACEUTICAL CO.**
**Shinjuku-ku, Tokyo 160-8515 (JP)**

(74) Representative: **Wablat, Wolfgang, Dr.Dr.**
**Patentanwalt,**
**Potsdamer Chaussee 48**
**14129 Berlin (DE)**

# (54) NOVEL SERINE PROTEASE INHIBITORY PROTEIN MT0039

(57) A gene and a protein participating in the control of serine protease activity and a method of efficiently evaluating an activity controlling agent for the protein are provided.

A DNA comprising the nucleotide sequence represented by SEQ ID NO:1; a serine protease inhibitory protein encoded by the DNA; a method of screening for an agent capable of binding to the protein with the use of the protein; an antisense nucleic acid to the DNA; and an antibody specific for the protein.

EP 1 433 848 A1

SEQUENCE LISTING

<110> Mochida Pharmaceutical Co., Ltd.
<120> Novel Serine protease inhibitor protein
<130> SAP-688-PCT
<150> JP 2001266004
<151> 2001-09-03
<160> 2
<170> PatentIn Ver. 2.1

<210> 1
<211> 1278
<212> DNA
<213> Homo Sapiens
<400> 1
atggactctc ttgttacagc aaacaccaaa ttttgctttg atctttttca agagataggc 60
aaagatgatc gtcataaaaa catatttttc tctcccctga gcctctcagc tgcccttggt 120
atggtacgct tgggtgctag aagtgacagt gcacatcaga ttgatgaggt actacacttc 180
aacgaatttt cccagaatga aagcaaagaa cctgaccctt gtctgaaaag caacaaacaa 240
aaagtgctgg ctgacagctc tctggaggga cagaaaaaaa cgacagagcc tctggatcag 300
caggctgggt ccttaaacaa tgagagcgga ctggtcagct gctactttgg gcagcttctc 360
tccaaattag acaggatcaa gactgattac acactgagta ttgccaacag gctttatgga 420
gagcaggaat tcccaatctg tcaggaatac ttagatggtg tgattcaatt ttaccacacg 480
acgattgaaa gtgttgattt ccaaaaaaac cctgaaaaat ccagacaaga ggttaacttc 540
tgggttgaat gtcaatccca aggtaaaatc aaggaactct tcagcaagga cgctattaat 600
gctgagactg tgctggtact ggtgaatgct gtttacttca aggccaaatg ggaaacatac 660
tttgaccatg aagacacggt ggatgcacct ttctgtctaa atgcgaatga aaacaagagt 720
gtgaagatga tgacgcaaaa aggcctctac agaattggct tcatagagga ggtgaaggca 780
cagatcctgg aaatgaggta caccaagggg aagctcagca tgttcgtgct gctgccatct 840
cactctaaag ataacctgaa gggtctggaa gagcttgaaa ggaaaatcac ctatgaaaaa 900
atggtggcct ggagcagctc agaaaacatg tcagaagaat cggtggtcct gtccttcccc 960
cggttcaccc tggaagacag ctatgatctc aattccattt tacaagacat gggcattacg 1020
gatatctttg atgaaacgag ggctgatctt actggaatct ctccaagtcc caatttgtac 1080
ttgtcaaaaa ttatccacaa aacctttgtg gaggtggatg aaaacggtac tcaggcagct 1140
gcagccactg gggctgttgt ctcggaaagg tcactacgat cttgggtgga gtttaatgcc 1200
aaccacccttt ttctcttttt cattagacac aacaaaaccc aaaccattct cttttatggc 1260

agggtctgct ctccttaa 1278

<210> 2
<211> 425
<212> PRT
<213> Homo sapiens
<400> 2

Met Asp Ser Leu Val Thr Ala Asn Thr Lys Phe Cys Phe Asp Leu Phe
1 5 10 15
Gln Glu Ile Gly Lys Asp Asp Arg His Lys Asn Ile Phe Phe Ser Pro
20 25 30
Leu Ser Leu Ser Ala Ala Leu Gly Met Val Arg Leu Gly Ala Arg Ser
35 40 45
Asp Ser Ala His Gln Ile Asp Glu Val Leu His Phe Asn Glu Phe Ser
50 55 60
Gln Asn Glu Ser Lys Glu Pro Asp Pro Cys Leu Lys Ser Asn Lys Gln
65 70 75 80
Lys Val Leu Ala Asp Ser Ser Leu Glu Gly Gln Lys Lys Thr Thr Glu
85 90 95
Pro Leu Asp Gln Gln Ala Gly Ser Leu Asn Asn Glu Ser Gly Leu Val
100 105 110
Ser Cys Tyr Phe Gly Gln Leu Leu Ser Lys Leu Asp Arg Ile Lys Thr
115 120 125
Asp Tyr Thr Leu Ser Ile Ala Asn Arg Leu Tyr Gly Glu Gln Glu Phe
130 135 140
Pro Ile Cys Gln Glu Tyr Leu Asp Gly Val Ile Gln Phe Tyr His Thr
145 150 155 160
Thr Ile Glu Ser Val Asp Phe Gln Lys Asn Pro Glu Lys Ser Arg Gln
165 170 175
Glu Val Asn Phe Trp Val Glu Cys Gln Ser Gln Gly Lys Ile Lys Glu
180 185 190
Leu Phe Ser Lys Asp Ala Ile Asn Ala Glu Thr Val Leu Val Leu Val
195 200 205
Asn Ala Val Tyr Phe Lys Ala Lys Trp Glu Thr Tyr Phe Asp His Glu
210 215 220
Asp Thr Val Asp Ala Pro Phe Cys Leu Asn Ala Asn Glu Asn Lys Ser

225 230 235 240
Val Lys Met Met Thr Gln Lys Gly Leu Tyr Arg Ile Gly Phe Ile Glu
245 250 255
Glu Val Lys Ala Gln Ile Leu Glu Met Arg Tyr Thr Lys Gly Lys Leu
260 265 270
Ser Met Phe Val Leu Leu Pro Ser His Ser Lys Asp Asn Leu Lys Gly
275 280 285
Leu Glu Glu Leu Glu Arg Lys Ile Thr Tyr Glu Lys Met Val Ala Trp
290 295 300
Ser Ser Ser Glu Asn Met Ser Glu Glu Ser Val Val Leu Ser Phe Pro
305 310 315 320
Arg Phe Thr Leu Glu Asp Ser Tyr Asp Leu Asn Ser Ile Leu Gln Asp
325 330 335
Met Gly Ile Thr Asp Ile Phe Asp Glu Thr Arg Ala Asp Leu Thr Gly
340 345 350
Ile Ser Pro Ser Pro Asn Leu Tyr Leu Ser Lys Ile Ile His Lys Thr
355 360 365
Phe Val Glu Val Asp Glu Asn Gly Thr Gln Ala Ala Ala Ala Thr Gly
370 375 380
Ala Val Val Ser Glu Arg Ser Leu Arg Ser Trp Val Glu Phe Asn Ala
385 390 395 400
Asn His Pro Phe Leu Phe Phe Ile Arg His Asn Lys Thr Gln Thr Ile
405 410 415
Leu Phe Tyr Gly Arg Val Cys Ser Pro
420 425

## Description

### FIELD OF THE INVENTION

The present invention relates to a novel serine protease inhibitory protein, a DNA encoding the protein and a recombinant vector, a transformant produced by using the vector, an antibody specific for the protein as well as diagnostic agents, a method for screening for pharmaceutical agents and the like by using the protein or the DNA encoding the protein.

### BACKGROUND OF THE INVENTION

Many reports demonstrating that proteases have important roles in various biological control mechanisms are submitted based on vigorous researches until now. Thus, proteases have important roles as a member in various biological control mechanisms from body level to cellular level, including the control of blood coagulation system as found in thrombin, the control of the expression of a digestive enzyme as found in chymotrypsin, the control of vasolidation as found in kallikrein, the control of apoptosis as found in caspase and cathepsin D and the like.

Now, an attempt such that the presence of a novel protease which has never reported will be confirmed and its biochemical properties and a physiological mode of action associated with it will be elucidated is noted. This elucidation is said to be very interested in the development of medicines since it is scientifically meaningful and also the relation between a novel protease and certain diseases can be clarified.

It is considered that proteases exhibit important roles in biological control mechanisms by specifically hydrolyzing its targeting protein. Generally it is fully expected that a protein is hydrolyzed irreversibly under physiological conditions and the so-called cascade reactions may occur starting from this hydrolysis. Therefore, it is presumed that such events that protease activity is exhibited unexpectedly or it is not exhibited at the proper time result in abnormal control in a series of biochemical reactions starting with the hydrolysis. So, it is easily guessed that the expression of protease activity per se is put under restrict control.

Activities of various serine proteases operating in coagulation system, fibrinolysis system, complement system and the like are controlled by proteins having serine protease inhibitory activities. Mechanism how to control the protease with a protease inhibitory protein is that protease and the protease inhibiting protein form reversibly or irreversibly an inactive complex and they are stabilized. For example, Kunitz-type inhibitors such as aprotinin and the like are reversible inhibitors, while serpins such as antithrombin and the like are irreversible inhibitors.

The above endogenous serine protease inhibitory proteins includes plasminogen activator inhibitor-1 and 2 (PAI-1, 2), kallistatin, neuroserpin, $\alpha$1-antitrypsin, $\alpha$1-antichymotrypsin (ACT), nexin, protein C inhibitor (PCI), $\alpha$2-antiplasmin ($\alpha$2-PI) and the like, in addition to antithrombin. Physiological functions of these serine protease inhibitors have been reported. For example, Suzuki et al., Thromobosis and Haemostasis, Vol. 61, pp. 337-342 (1989) describes that a serine protease inhibitor inhibits various factors participating in blood coagulation and fibrinolysis so that blood coagulation and fibrinolysis systems are controlled. Buisson et al., The FASEB Journal, Vol. 12, pp. 1683-1691 (1998) describes that a serine protease inhibitor modulates the growth, the migration and the invasion of cells directly or indirectly so that immune and nervous systems are controlled and that the serine protease inhibitor is participated in the growth, the migration and the invasion of tumor cells. Further, Uhrinetal., J. Clin. Invest., Vol. 106, pp. 1531-1539 (2000) describes that a serine protease inhibitor is participated in the differentiation and the maturation of germ cells so that a germ system is controlled. Egbert et al., Blood, Vol. 86, pp. 4007-4024 (1995) describes that a serine protease inhibitor is participated in the decomposition of extracellular matrix components so that reconstitution, development and differentiation of tissues are controlled.

WO 01/74851 describes the sequence (named "NOV1") wherein deletion of 30 amino acids (corresponding to the residue from 72 to 101 a.a. of MT0039) and substitution of two amino acids have occurred in the amino acid sequence of MT0039. It is assumed that NOV1 may have a SCCA (sruamous cell carcinoma antigen) family-like function based on its homology with SCCA, but such a function is not demonstrated therein. WO 01/81363 describes the sequence (SEQ ID NO:59) wherein deletion of 31 amino acids (corresponding to the residue from 62 to 92 a.a. of MT0039) and substitution of one amino acid have occurred in the amino acid sequence of MT0039. It is assumed that this sequence may have a SCCA family-like function based on its homology with SCCA, but such a function is not demonstrated therein. [Online], [July 25, 2001], Internet <URL:http://www.gsc.riken.go.jp/e/FANTOM/>clone ID 2300003F07 describes the sequence having a 83% homology with the amino acid sequence of MT0039 (FANTOM clone ID 2300003F07) . It describes that this sequence has a serpin-like domain and that this sequence is homologous with SCCA, but its function is not demonstrated therein. Askew, Y. et al., JBC, Vo. 276, pp. 49320-49330 (2001) describes the amino acid sequence (named "SERPINB12") wherein the deletion of 20 amino acids (corresponding to the residue from 82 to 101 a.a. of MT0039) has occurred in the amino acid sequence of MT0039.

According to the above background, it is desired to find out a new biomolecule capable of inhibiting a protease activity and a gene encoding it, to clarify its activity and to develop a method of screening for medicines based on a new mechanism involved in the above inhibition, an activity controller, a specific antibody or an antisense nucleic acid.

## SUMMARY OF THE INVENTION

The present invention provides a novel gene (mt0039) isolated from human thymus and a serine protease inhibitory protein (MT0039) encoded by said gene. And, the present invention provides a recombinant vector including said DNA, a host cell transformed with said gene, an antibody against said protein, an antisense nucleic acid and a screening method.

<Nucleic acid>

The present invention provides the gene mt0039 encoding MT0039 as fully described below. Specifically, the gene mt0039 is a DNA encoding a serine protease inhibitory protein comprising the amino acid sequence represented by SEQ ID NO:2 and it includes a cDNA represented by SEQ ID NO:1 and a genomic DNA that said cDNA comes from.

Although the gene mt0039 can be isolated and identified from a cDNA library from human thymus and cDNA libraries from various organs where the expression of the gene is confirmed, the gene may be a DNA obtained by cloning using a genetic engineering technique such as a standard hybridization or a chemical synthetic technique such as phosphoramidate method based on the sequence as disclosed herein. The form of the gene may be a cDNA, a genomic DNA and a chemically synthesized DNA, however not limited thereto. The DNA of the present invention may be a single-strand DNA. Alternatively, it may bind to a DNA or an RNA having the sequence complementary thereto to form a double- or triple-strand. The DNAmaybe labeled with an enzyme such as horseradish peroxidase (HRPO); a radioisotope; a fluorescent substance; a chemiluminescent substance; and the like.

If the nucleotide sequence of mt0039 is provided, a sequence of an RNA and sequences of a complementary DNA and RNA are only one-sidedly determined. Therefore, it should be understood that the present invention also provides an RNA corresponding to the DNA of the present invention as well as a DNA and an RNA having the sequence complementary to the DNA of the present invention. "DNA" and "polynucleotide" are interchangeably used herein.

The DNA of the present invention also includes a DNA having an identity of at least 90% with the nucleotide sequence represented by SEQ ID NO:1.

Variations of the nucleotide sequence represented by SEQ ID NO:1 are acceptable as long as a protein encoded by said DNA is a serine protease inhibitory protein, particularly a serine protease inhibitory protein having an activity of enhancing the induction of apoptosis by tumour necrosis factor. A DNA sequence partially modified by, for example, the presence of plural codons encoding the same amino acid residue due to the degeneracy of codon; and various artificial treatments such as site-specific mutation, random mutation by treating with a mutagen, mutation, deletion, linkage and the like of a DNA fragment by digesting with a restriction enzyme are included within the present invention as long as the DNA mutant has an identity with the DNA represented by SEQ ID NO:1 of at least 90% and encodes a serine protease inhibitory protein having an activity of enhancing the induction of apoptosis by tumour necrosis factor even if its sequence is different from the DNA sequence represented by SEQ ID NO:1.

The DNA mutant is acceptable as long as it has an identity with the DNA sequence represented by SEQ ID NO:1 of at least 90%, preferably at least 96%, more preferably at least 98%, even more preferably at least 99%. The identity in DNA sequence can be analyzed by BLAST (J. Mol. Evol., Vol. 36, pp. 290-300 (1993); J. Mol. Biol., Vol. 215, pp. 403-410 (1990)). The term "hybridizable" means that a DNA is hybridizable with the nucleic acid represented by SEQ ID NO: 1 by southern hybridization under stringent conditions. For example, if a probe labeled with DIG DNA Labeling kit (Cat No. 1175033 of Roche Diagnostics) is used , the hybridization is conducted in a DIG Easy Hyb solution (Cat No. 1603558 of Roche Diagnostics) at the temperature of, for example, 32°C (preferably 37°C, more preferably 42°C) and the membrane is washed in, for example, 0.5 x SSC solution containing 0.1% (w/w) of SDS at 50°C (preferably 65°C) (note: 1 x SSC is 0.15M NaCl and 0.015M sodium citrate).

It is believed that the specific expression of the DNA comprising the nucleotide sequence represented by SEQ ID NO:1 in kidney, thymus, placenta, uterus, testes, prostate and brain is demonstrated and therefore the DNA or its partial fragment is useful as a specific probe for diseases in the above organs.

The DNA of the present invention can be used to commercially produce MT0039. And, the DNA can be used for testing an expression status of the protein of the present invention in a tissue by labeling with an enzyme or the like. That is, an expression amount of mRNA as an index of an expression amount of the protein of the present invention in a cell is demonstrated by using the DNA as a probe so that a cell and cultivating conditions of the cell suitable for thepreparation of the protein of the present invention can be determined. In addition, a disease associated with the protein of the present invention can be diagnosed.

Further, an abnormality or polymorphism on the nucleic acid sequence can be tested or diagnosed by any method such as PCR-RFLP (Restriction fragment length polymorphism) method, PCR-SSCP (Single strand conformation polymorphism), sequencing method and the like, using a part of the DNA of the present invention as a primer.

And, the DNA of the present invention can be used in gene therapy of a disease in which the expression or the activity of the protein of the present invention is lost, by transfecting the DNA of the present invention into an in vivo cell.

The DNA of the present invention is very useful in the preparation of a transformant, the production of a recombinant protein MT0039 using said transformant and the screening of a compound specifically controlling the expression of MT0039.

The transformant of the present invention can be produced according to a method known for those skilled in the art. For example, the DNA of the present invention can be incorporated into a suitable host cell using any one of vectors commercially available or easily obtained by those skilled in the art. Then, the expression of the gene mt0039 within a host cell can be suitably controlled by placing the gene mt0039 under the influence of an expression control gene, typical examples of which are a promoter and an enhancer. This technique is suitable for being used in the production of the protein MT0039 using the transformed host cell as well as the investigation of mechanisms how to control the expression of the gene mt0039 and the screening of an agent capable of controlling the expression of the gene.

For example, by contacting any test substances with a cell transformed with the vector containing the gene mt0039 under suitable conditions, an agent capable of promoting or inhibiting the expression of the gene mt0039 can be screened or evaluated among the test substances.

By using the DNA of the present invention in combination with a known technique, a transgenic animal can be produced from a suitable animal such as mouse or the like. And, it is possible to produce the so-called knockout non-human animal in which a orthologue gene corresponding to mt0039 is destroyed in the animal by using the mt0039 gene of the present invention. By introducing the human mt0039 of the present invention into an animal whose endogenous gene is destroyed, a model animal having only human mt0039 can be produced.

By observing the above transgenic animal, especially an animal expressing a large amount of the gene mt0039 or the protein MT0039 of the present invention or an animal from which the gene mt0039 of the present invention is omitted, it is possible to identify physiological functions of the gene mt0039 or the protein MT0039. Further, this model animal is useful in development and evaluation of a drug targeting the human MT0039 introduced in said model. That is, it becomes possible to screen for an agent specifically acting on the gene mt0039 or the protein MT0039 or supplementing its function at *in vivo* level. The thus-screened agent is expected to be a drug affecting a biological control system in which MT0039 specifically functions. Especially since a specific expression of the gene or the protein of the present invention is observed in testes, compounds obtained by screening for the above transformant or transgenic animal are expected as effective therapeutic or preventive agents for diseases caused by a dysfunction of testes.

The so-called antisense nucleic acid capable of inhibiting the biosynthesis of MT0039 at a nucleic acid level *in vivo* is useful. The antisense nucleic acid means a nucleic acid which binds to DNA or RNA involved in carrying a genetic information during either of a transcription stage from a genome region to a pre-mRNA essential for the production of mRNA encoding MT0039, a processing stage from the pre-mRNA to a mature mRNA, a stage of passing through a nuclear membrane or a translation stage into a protein so as to affect the normal stream of the transmission of the genetic information and thereby to control the expression of the protein. It may comprises a sequence complementary to the entire nucleic acid sequence of the gene mt0039 or either part of the sequence. Preferably, it is anucleic acid (including DNA and RNA) comprising a sequence corresponding to or complementary to the nucleic acid sequence represented by SEQ ID NO:1. When mRNA transcripted from the genome region contains an intron structure or a untranslated region at 5' or 3' -terminal, an antisense nucleic acid corresponding to or complementary to the sequence of the untranslated region will have functions equivalent to those of the antisense nucleic acid of the present invention.

The antisense nucleic acid of the present invention includes a DNA and an RNA as well as all of derivatives similar to the DNA and the RNA in configuration and functions. Example of the antisense nucleic acid includes a nucleic acid having any other substance bound at 3'- or 5'-terminal, a nucleic acid wherein at least one of bases, sugars and phosphates of the oligonucleotide is substituted or modified, a nucleic acid having a non-naturally occurring base, sugar or phosphate, a nucleic acid having a backbone other than the sugar-phosphate backbone and the like. These nucleic acids are suitable as derivatives in which at least one of a nuclease resistance, a tissue selectivity, a cell permeability and a binding activity is improved. That is, the form of the nucleic acid is not limited as long as the nucleic acid can inhibit the activity and the expression of MT0039.

And, the antisense nucleic acid having the nucleotide sequence complementary to the nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a ribosome binding site, a capping site and a splicing site, i.e. an antisense nucleic acid having the sequence complementary to that of these sites is also preferable since generally it canbe expected to be very effective in inhibiting the expression.

In order to make the above antisense nucleic acid introduced into a cell and act efficiently, it is suitable that the length of the antisense nucleic acid of the present invention is 15 to 30 bases, preferably 15 to 25 bases, more preferably 18 to 22 bases.

The effect of the antisense nucleic acid of the present invention in controlling the expression can be evaluated by a known method, for example, by preparing an expression plasmid comprising a reporter gene such as luciferase and the like linked to the DNA containing an expression control region, a 5'-untranslated region, a region near a translational initiation site or a part of a translated region of the gene of the present invention, adding a candidate under an environment where the gene of the present invention is transcripted or translated as in a system of *in vitro* transcription (ribo max systems; Promega) together with *in vitro* translation (Rabbit Reticulocyte Lysate Systems; Promega) and determining an expression amount of the reporter gene.

Since the antisense nucleic acid of the present invention can inhibit the expression of MT0039 in vivo, it is expected that the antisense nucleic acid is useful as an effective therapeutic or prophylactic agent for diseases associated with MT0039.

<Protein MT0039>

A protein encoded by mt0039 is MT0039 comprising the amino acid sequence represented by SEQ ID NO:2.

MT0039 has a structure which is expected to be a serine protease inhibitory domain in view of an analytical result based on the amino acid sequence and therefore, it is anticipated to be one of serine protease inhibitors.

Within an amino acid sequence of a substrate to be cleaved with protease, an amino acid residue newly produced by cleavage at C-terminal is called "P1". Residues elongating towards N-terminal from P1 are called P2, P3, P4 and so one. And, residues elongating towards C-terminal are called P1', P2' and so on. It has been known that serpin in which the sequence at an active center (P1-P1') is Arg-X has an inhibitory activity on trypsin type serine protease. The residue estimated to be an active center in MT0039 is Arg390 - Ser391 so that MT0039 is also expected to inhibit trypsin-like serine protease activity. Now, the present inventors clarified the gene sequence of a novel serine protease inhibitory protein and further clarified its function, its specificity and its gene expression pattern, leading to the present invention.

As the result of homology searching with known proteins, the amino acid sequence of MT0039 was homologous with mouse putative (GenBankAccession: BAB26028) at the level of 83%; human SQUAMOUS CELL CARCINOMA ANTIGEN 2 (GenBank Accession: SCC2_HUMAN) at the level of 62%; and human SQUAMOUS CELL CARCINOMA ANTIGEN 1 (GenBank Accession: SCC1_HUMAN) at the level of 62%.

While, as to the mouse putative (GenBank Accession: BAB26028), FANTOM data base (http://www.gsc.riken.go.jp/e/FANTOM/, FANTOM clone ID 2300003F07) describes that it has a serpin-like domain and it is homologous with SQUAMOUS CELL CARCINOMA ANTIGEN 2 (leupin; Bartuski et al., Genomics, Vol. 54, pp. 297-306 (1998) ), but it does not suggest nor demonstrate how to function as a protein. And, the residue estimated to be an active center in the mouse putative (GenBank Accession: BAB26028) is irregular since it is Lys-Ala and therefore it is unknown what protease is inhibited.

In NOV1 as described in WO 01/74851, SEQ ID NO:59 as described in WO 01/81363 and SERPINB 12, a 72 to 101 a.a. residue, a 62 to 92 a.a. residue and a 82 to 101 a.a. residue are deleted from MT0039, respectively. Considering the 3-dimensional structure of other serpins such as $\alpha$1-antitrypsin, a region containing such a deletion corresponds to a loop region linking $\alpha$-helix regions. MT0039 is characterized by that the loop region is longer than those of other serpins and this characteristic loop is possibly associated with functional characteristics of MT0039. Accordingly, either protein deleting this loop region is considered to be functionally distinguishable from MT0039.

From the detailed analysis of the structure and activity of MT0039, it is demonstrated in the present invention that the protein of the present invention has a serine protease inhibitory activity. Physical functions of MT0039 are also clarified in the present invention. Thus, according to the present invention, MT0039 is found to be a serine protease inhibitory protein having an activity of enhancing the induction of apoptosis by tumor necrosis factor.

It was demonstrated that the gene mt0039 was specifically expressed in kidney, thymus, placenta, uterus, testes, prostate and brain (Fig. 2). This results are clearly different from the expression profile of SERPINB12, suggesting that MT0039 and SERPINB12 are functionally distinguishable each other. It has been known that serine proteases present in placenta, uterus and testes are participated in controlling the maturation of germ cells, the growth of follicles, the ovulation, the acrosomal reaction, the implantation or placental functions so that MT0039 is expected to control a reproductive system by controlling its activity.

Therefore, it is expected that MT0039 can be used as medicines for diseases associated with dysfunction of reproductive organs such as cancers, infertility and abnormal pregnancy as well as for contraception. And, agents inhibiting the binding between MT0039 and its targeting serine protease are valuable as pharmaceutical candidates. Thus, MT0039 may be a very important target during the screening for the above agents.

It was demonstrated that MT0039 inhibits trypsin, plasmin and cathepsin G (Example 8). It has been known that trypsin, plasmin and cathepsin G either activates a matrix metalloprotease. On the other hand, it has been known that the thus-activated matrix metalloprotease causes the acceleration of inflammatory reaction, the remodeling of tissues, the fibrosis of tissues, the vascularization and the like. Therefore, MT0039 is expected to prevent the progress of diseases including inflammatory diseases such as nephritis, hepatitis, pneumonitis, pancreatitis, ARDS and the like; diseases associated with the remodeling and the fibrosis of tissues such as arteriosclerosis, myocardial infraction, pulmonary fibrosis, wound and the like; diseases associated with the vascularization such as cancers and the like. And, it has been known that plasmin enhances a fibrinolytic system. Therefore, MT0039 is expected to treat for diseases where a fibrinolytic system is enhanced such as disseminated intravascular coagulation syndrome and the like. Further, it has been known that cathepsin G produces angiotensin II in kidney, that cathepsin G produces APP found in Alzheimer brain and that cathepsin G activates immunocompetent cells. Therefore, MT0039 is expected to prevent the progress of diseases such as hypertension, Arzheimer diseases, irritable bowel syndrome and the like.

On the other hand, agents preventing the formation of a complex of MT0039 and serine protease increases a plasmin activity so that they are expected to prevent the progress thrombosis such as cerebral infarction, myocardial infarction, phlebothrombosis, pulmonary embolism and the like. And, these agents are expected to prevent the progress of cancers and infections by increasing a cell immunity.

It was demonstrated that apoptosis is accelerated by transfection of MT0039 into cells (Example 6). Therefore, the DNA of the present invention can be used to eliminate cells in vivo by introducing said DNA into them. For example, caner cells can be specifically eliminated by specifically expressing the DNA of the present invention on the cells. Cells infected with virus can be specifically eliminated by specifically expressing the DNA of the present invention on the cells.

And, apoptosis can be prevented by inhibiting the formation of a complex of MT0039 and serine protease. Therefore, agents preventing the formation of such a complex are expected to be used as therapeutic agents for diseased associated with apoptosis such as cerebral infarction, myocardial infarction, shock, fulminant hepatitis, HIV and the like.

A protein having an amino acid sequence wherein substitution, deletion and/or addition (insertion) of one or more amino acids has occurred in the amino acid sequence of the protein represented by SEQ ID NO:2 is included within the scope of the present invention as long as this protein is a serine protease inhibitory protein having an activity of enhancing the induction of apoptosis by tumour necrosis factor.

Side chains of amino acid residues which are constitutional elements of a protein are different in terms of hydrophobicity, charge, size and the like, but they are known to have several highly conservative relationships since they do not substantially affect a three-dimensional structure (also called as configuration) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (Gln) ; aspartic acid (Asp) and asparagine (Asn); cysteine (Cys) and threonine (Thr); Thr and serine (Ser) or Ala; lysine (Lys) and arginine (Arg); and the like. Since Ala, Val, Leu, Ile, Pro, methionine (Met), phenylalanine (Phe), tryptophane (Trp), Gly and Cys are classified as non-polar amino acids, they are understood to have similar properties to each other. Non-charged polar amino acids include Ser, Thr, tyrosine (Tyr) , Asn and Gln. Acidic amino acids include Asp and Glu. Basic amino acids include Lys, Arg and histidine (His). Even if the conservation as defined above is lost, many mutants maintaining functions essential for the protein (in the present invention, the function as a serine protease inhibitory protein) are known for those skilled in the art. Further, in several similar proteins conserved between different species, it is recognized that they maintain essential functions even if several amino acids are deleted or inserted concentratedly or scatteringly.

Accordingly, a protein resulting from substitution, insertion, deletion and/or addition of one or more amino acids in the amino acid sequence represented by SEQ ID NO:2 are included within the scope of the present invention as long as it has a function substantially equivalent to that of MT0039. The term "functionally equivalent" means that a protein in question is a serine protease inhibitory protein having an activity of enhancing the induction of apoptosis by tumour necrosis factor. The term "serine protease inhibitory protein" as used herein means a protein capable of controlling the activity of serine protease by binding to serine protease. It should be understood that the change in strength of the binding between the protein and serine protease, the change in molecular weight of the protein due to the difference in sugar chain binding or the like are acceptable as long as an ability of binding to serine protease or a serine protease inhibitory activity, and/or an activity of enhancing the induction of apoptosis by tumour necrosis factor are qualitatively equivalent. The term "qualitatively equivalent in serine protease inhibitory activity" means that a protein in question has at least trypsin, plasmin and cathepsin G inhibitory activities. More preferably, a protein in question has thrombin, kallikrein, activated protein C, tissue plasminogen activator (t-PA), activated factor X (FXa), urokinase-type plasminogen activator (u-PA), granzyme, chymotrypsin, neutrophil elastase, cathepsin K and cathepsin L inhibitory activities weaker than trypsin, plasmin and cathepsin G inhibitory activities, in addition to the above trypsin, plasmin and cathepsin G inhibitory activities. The term "weaker" as used herein means that each activity corresponds to 30% or less, preferably 10% or less, of trypsin, plasmin and cathepsin G inhibitory activities. The term "an activity of enhancing the induction of apoptosis by tumour necrosis factor" means that the induction of apoptosis by tumour necrosis factor is enriched in cultured mammalian cells (for example, HeLa cells) transformed by a recombinant vector including the mt0039 gene and expressing the MT0039 protein as compared with non-transformed cells. The above activity can be confirmed by the method as described in Example 6. A tumour necrosis factor (TNF) used upon confirming an activity of MT0039 in enhancing the induction of apoptosis by tumour necrosis factor is preferably TNF-α. When cultured mammalian cells where MT0039 is to be expressed are cells from human, a human TNF-α is preferably used. When cultured mammalian cells where MT0039 is to be expressed are cells from mouse, a mouse TNF-α is preferably used. However, the nature of TNF-α is not particularly limited as long as TNF-α can induce apoptosis in cultured cells to be used.

The changes in amino acids are found in the nature such as mutants caused by gene polymorphism and the like. Further, it can be produced artificially according to a known method for those skilled in the art, for example, mutagenesis using a mutagene such as NTG and site-directed mutagenesis using various recombinant gene techniques. The site and the number of the mutation of amino acids are not particularly limited as long as the resultant mutant protein is a serine protease inhibitory protein having an activity of enhancing the induction of apoptosis by tumour necrosis factor. The mutation number is generally within several tens of amino acids, preferably within 10 amino acids, more preferably within 1 or several amino acids.

And, a serine protease inhibitory protein comprising an amino acid sequence homologous with the amino acid sequence of SEQ ID NO:2 at a level of at least 90%, more preferably at least 95%, further preferably at least 98% and having an activity of enhancing the induction of apoptosis by tumour necrosis factor is within the present invention. For judging the homology of amino acid sequences, BLAST (J. Mol. Evol., Vol. 36, pp. 290-300 (1993) ; J. Mol. Biol., Vol. 215, pp. 403-10 (1990) ) can be used.

Of course, the above protein may be in the form of a salt within the general knowledge of those skilled in the art. And, the protein of the present invention includes a single protein and proteins transformed into various forms including a fused protein with any other different protein. For example, it is thought that the protein can be processed according to various methods known for those skilled in the art such as various chemical modifications on the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It shouldbe understood that all of the above proteins are included within the scope of the present invention as long as said proteins function as inhibitory proteins. Asn 66, Asn 107, Asn 238, Asn 375 and Asn 411 in MT0039 are presumed to be sites to which N-sugar chains are bound.

The protein or its partial peptide of the present invention can be used in screening for an agent capable of controlling the activity of said protein. The thus-screened compounds and the like are expected to be useful as effective therapeutic or preventive agents for diseases associated with the protein of the present invention.

<Antibody>

Further, the present invention provides an antibody bound to MT0039. The antibody of the present invention is an antibody specifically recognizing this protein as an antigen. It includes a monoclonal antibody and/or a polyclonal antibody. And, it may be an antibody belonging to any one of five classes (IgG, IgA, IgM, IgD and IgE) classified by the structure, physical-chemical properties and immunological properties of immunoglobulins or either subclass classified by the type of H chain. Further, it may be a fragment such as $F(ab')_2$ produced by digesting an immunoglobulin with, for example, pepsin, Fab producedby digesting an immunoglobulin with papain and the like, a chimeric antibody, or a humanized antibody. The antibody is useful in investigation or clinical therapy of MT0039, clinical therapy of diseases caused by MT0039 and the like. For example, the presence of absence of the protein of the present invention in blood, body fluid, tissue or cell, its amount, the abnormality of its structure and the like can be detected and diagnosed using western blotting, immunoprecipitation, ELISA and the like.

## DISCLOSURE OF THE INVENTION

Embodiments of the present invention will be described below. As to principles and uses of genetic engineering techniques, biochemical techniques using proteins, cells or animals as well as various devices such as a chromatography and a peptide synthesizer, their applications are not limited otherwise specified. Since techniques available by those skilled in the art, their principles and their uses can be applied to the present invention, it should be understood that they are not limited by the following description.

<Nucleic acid>

The method for obtaining the DNA of the present invention from a DNA library includes a method comprising screening a suitable genomic DNA library or cDNA library according to a screening method such as a screening method via hybridization and an immunoscreening method using an antibody, amplifying a clone having the desired DNA and cleaving the DNA with a restriction enzyme or the like. In the screening method via hybridization, the hybridization can be conducted for any cDNA library using the DNA having the nucleotide sequence represented by SEQ ID NO:1 or a part thereof labeled with $^{32}P$ or the like as aprobe according to a knownmethod (see, for example, Maniatis, T. et al., Molecular Cloning, a Laboratory Manual, Cold Spring Harbor Laboratory, New York (1982)) . The antibody used in the immunoscreening method may be an antibody of the present invention as described below. The novel DNA of the present invention may be obtained by PCR (Polymerase Chain Reaction) using a genomic DNA library or a cDNA library as a template. PCR is conducted for any DNA library according to a known method (see, for example, Michael, A.I. et al., PCR Protocols, a Guide to Methods and Applications, Academic Press (1990) ) using a sense or antisense primer prepared based on the nucleotide sequence of SEQ ID NO:1, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library can be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.

The DNA of the present invention can be prepared based on the sequence as disclosed herein by a chemical synthetic technique such as phosphoramidite method.

The recombinant vector including the DNA of the present invention may have any form such as a cyclic form or a linear form. The recombinant vector may have any other nucleotide sequence in addition to the entire or a part of the DNA of the present invention, if necessary. "A part" means, for example, a DNA encoding a partial peptide of the protein of the present invention. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding other polypeptide, a polyA addition sequence, a splicing sequence, a replication origin, a nucleotide sequence of the gene acting as a selective marker and the like. One preferable example of the recombinant vector of the present invention is an expression vector.

In the gene recombination, it is possible to add a translational start codon or a translational stop codon to the DNA of the present invention using a suitable synthetic DNA adapter, or to newly produce or delete a suitable restriction site within the nucleotide sequence. This is the technique routinely conducted by those skilled in the art. Such a processing can be suitably and easily conducted based on the DNA of the present invention.

As the vector including the DNA of the present invention, a suitable vector is selected and used depending on the type of a host to be used. The vector may be a plasmid. Alternatively, various viruses may be used, non-limiting examples of which include bacteriophage, baculo virus, retro virus, vaccinia virus and the like. Thus, various elements generally used in genetic engineering techniques may be added to the gene of the present invention and alternatively the gene of the present invention may be altered according to the general genetic engineering technique. It should be understood that elements and technique disclosed herein are not exclusive and elements and technique available by those skilled in the art are used without particular limitation.

The gene of the present invention can be expressed under the control of a promoter sequence inherent to said gene. Using the expression system, an agent promoting or suppressing the transcription of the gene of the present invention can be efficiently screened. Any other suitable expression promoter can be used by linking it to the promoter sequence inherent to said gene upstream of the gene of the present invention or replacing it with the promoter sequence. In this case, the promoter may be selected depending on a host or an object of expression. For example, if a host is E. coli, a T7 promoter, a lac promoter, a trp promoter, a $\lambda$PL promoter or the like can be used. If a host is a yeast, a PH05 promoter, a GAP promoter, an ADH promoter or the like can be used. If a host is an animal cell, a promoter from SV 40, a retro virus promoter, an elongation factor 1$\alpha$ promoter or the like can be used. These lists are not exclusive.

A method for introducing the DNA into a vector is known (see J. Sambrook et al., Molecular Cloning, a Laboratory Manual 2nd ed., Cold Spring Harbor Laboratory, New York (1989)) . That is, each of the DNA and the vector may be digested with suitable restriction enzymes and the resultant fragments may be ligated with a DNA ligase.

The antisense nucleic acid can be prepared according to a known method (see, for example, edited by Stanley T. Crooke and Bernald Lableu, in Antisense Research and Applications, published by CRC Publisher of Florida (1993)). If DNA and RNA are native, the antisense nucleic acid of the present invention can be obtained by synthesizing in a chemical synthesizer or conducting PCR using the mt0039 gene as a template. Alternatively, a part of derivatives such as methyl phosphonate type and phosphorothioate type can be synthesized in a chemical synthesizer (for example, Expedite Model 8909; Applied Biosystems Japan) . Then, such a derivative may be synthesized according to a manual attached to the chemical synthesizer and the thus-synthesized product may be purified by HPLC using a reverse phase chromatography or the like, thereby the antisense nucleic acid can be obtained.

When the antisense nucleic acid is used as a diagnostic probe, it is labeled with a radioisotope, an enzyme, a fluorescent substance, a luminescent substance or the like according to a known method. Subsequently, a DNA or an mRNA is prepared from a specimen according to a known method and it is used as a test substance. This test substance is reactedwith the labeledprobe and then the product is washed to remove the labeled probe unreacted. If the test substance contains the gene mt0039 or RNA, the antisense nucleic acid is bound thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending it in a suitable solvent. Alternatively, it may be used after encapsulating in a liposome or introducing into a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, abase, a stabilizer, a preservative, a solubilizing agent, an excipient, a buffer and the like.

When the DNA, the antisense nucleic acid or the recombinant vector of the present invention is used in the above dosage form, its administration method and its dose can be selected depending on the age and the sex of a patient, the type and the severity of the disease. That is, it may be administered in an amount suitable for improving conditions by a suitable method selected fromoral, inhalation, transdermal, eye dropping, intravaginal, intraarticular, intrarectal, intravenous, topical, intramuscular, subcutaneous and intraperitoneal administrations.

<mt0039 gene transferred non-human animal>

The present invention provides a mt0039 gene transferred non-human animal. The mt0039 gene transferred non-human animal includes transgenic non-human animals and knockout non-human animals. The mt0039 gene transferred non-human animal is characterized by that the expression of the protein of the present invention in terms of its degree, its time, its site and the like is controlled by artificially introducing the gene encoding the protein of the present invention on a chromosome of the animal. Non-limiting example of non-human animals includes cattle, sheep, goat, porcine, mouse, horse, chicken and the like. Among the non-human animals, non-human mammalian animals are preferable.

By using the gene mt0039 of the present invention, a transgenic non-human mammalian animal can be produced. The transgenic non-human mammalian animal can be produced according to a routine method conventionally used in the production of transgenic animals (see, for example, "Experimental Manual of Genesis, published by Kodansha Scientific Ltd., editedbyMotoya KATSUKI under supervision of Tatsuji NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.

Specifically, in case of a transgenic mouse, a DNA constructed such that the mt0039 gene can be expressed is directly injected into a pronucleic oosperm obtained from a normal C57Black/6 mouse. More specifically, a construct is prepared by introducing the mt0039 gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.

The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplanted offspring and confirmed whether or not the mt0039 gene is introduced by PCR or southern blotting, thereby a transgenic mouse is obtained.

The so-called "knock-out mouse" can be produced based on the nucleotide sequence of the mt0039 (or a mouse homologous gene of mt0039). The term "knock-out mouse" used herein means a mouse in which a mouse gene homologous with the gene of the present invention is knocked out (inactivated). The knock-out mouse can be produced by, for example, a positive-negative selection method via homologous recombination (see, for example, US patent Nos. 5, 464, 764, 5, 487, 992 and 5, 627, 059; Proc. Natl. Acad. Sci. USA, Vol. 86, pp. 8932-8935 (1989) ; Nature, Vol. 342, pp. 435-438 (1989)). Such a knock-out mouse is one embodiment of the present invention.

Recently, the production of clone animals by nuclear transplantation in medium or large animals becomes possible. In this connection, transgenic and knockout animals have been practically produced using this technique. That is, a somatic cell or a germinal cell is subj ected to homologous recombination based on the nucleotide sequence of the mt0039 (or a homologous gene of mt0039 in each animal) in the same way as that applied to ES cells and then a nucleus is obtained from the resultant cell and used to obtain a cloned animal. This animal is a knock-out animal in which the mt0039 gene (or a homologous gene of mt0039 in each animal) is lost. Or, the mt0039 gene (or a homologous gene of mt0039 in each animal) is introduced in any cell of any animal and then the resultant nucleus is used to obtain a clone animal, thereby a transgenic animal can be produced. Such a knock-out non-human animal and a transgenic non-human animal are one embodiment of the present invention irrespective of its species.

<Protein>

The protein of the present invention can be prepared from various organs naturally expressing said protein. Alternatively, it can be chemically synthesized in a peptide synthesizer (for example, Peptide Synthesizer Model 433A; Applied Biosystems Japan) or it can be produced by recombination methodusing a suitable host cell selected fromprokaryotic cells and eukaryotic cells. However, a genetic engineering technique and a recombinant protein produced thereby are preferable in view of purity.

A host cell to be transformed using the recombinant vector described in the previous section is not limitative. Many cells of low organisms available in genetic engineering techniques, typical examples of which are E. coli, B. subtilis and S. cerevisiae; and animal cells, typical examples of which are insect cell, COS7 cell, CHO cell and HeLa cell, can be used in the present invention.

The transformant of the present invention can be obtained by transforming a suitable host cell using the recombinant vector of the present invention. As the method of introducing the recombinant vector described in the previous section into a host cell, some methods are known, such as an electroporation, a protoplast method, an alkali metal method, a calcium phosphate precipitation method, a DEAE-dextran method, a microinjection method, a method using virus particles and the like (see "Handbook of Genetic Engineering", Special Issue of Experimental Medicines, published by Yodosha Co., Ltd. (March 20, 1991)). Either method may be used.

For preparing the present protein by a genetic engineering technique, the above transformant is cultured to obtain a culture mixture followed by purify the present protein. The transformant can be cultured according to a standard method. Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, electrophoresis, gel filtration chromatography, ion-exchange chromatography, hydrophobic chromatography and various affinity chromatographies including antibody chromatography, chromatofocusing, adsorption chromatography, reverse phase chromatography and the like. If necessary, HPLC systems or the like may be used to conduct several methods in a suitable order.

It is possible to express the protein of the present invention as a fusion protein with any other protein or tag such as glutathion S transferase, Protein A, hexahistidine tag, FLAG tag and the like. The thus-expressed fusion protein may be cleaved with a suitable protease such as thrombin, enterokinase and the like. This may be more effective for the preparation of the protein. For purifying the protein of the present invention, conventional methods may be suitably combined. Especially if the protein is expressed in the form of a fusion protein, it is preferable to purify according to a method characteristic to such a form.

One of methods for preparing the present protein by a genetic engineering technique is the synthesis of a cell-free system using a recombinant DNA molecule (J. Sambrook et al., Molecular Cloning 2nd ed. (1989)).

The protein of the present invention can be used as an antigen for the preparation of an antibody and in screening for an agent capable of binding to said protein or controlling an activity of said protein and therefore, it is useful.

When the expressed MT0039 is present in a periplasm or a cytoplasm of the transformant, the transformant suspended in a suitable buffer is subjected to a treatment such as an ultrasonic treatment, a freeze-thawing treatment, a treatment with lysozyme or the like to destroy a cell wall and/or a cell membrane and further subjected to centrifugation, filtration or the like to obtain a fraction containing the protein of the present invention. Next, this fraction is solubilized with a suitable surfactant to prepare a crude solution, from which the desired protein can be isolated and purified according to a routine method.

<Antibody>

The antibody of the present invention may be polyclonal or monoclonal. Either antibody can be obtained by referring to a known method (see, for example, "Experimental Procedures in Immunology", edited by Japan Society for Immunology, published by Japan Society for Immunology), as describe below in brief.

For obtaining a polyclonal antibody, an animal is inoculated with the protein of the present invention as an immunizing antigen and if necessary a suitable adjuvant such as Freund's complete adjuvant (FCA), Freund's incomplete adjuvant (FIA) and the like. If necessary, a booster at an interval of 2 to 4 weeks may be conducted. After the booster, an anti-serum is obtained by taking a blood sample. The protein of the present invention used as an antigen is that obtained in any method as long as it has a purity sufficient to be usable in the preparation of an antibody. A partial polypeptide of the protein of the present invention may be suitably used as an immunizing antigen. If the polypeptide used as an immunizing antigen is a low-molecular weight polypeptide, i.e. apolypeptide comprising about 10 to 20 amino acids, it may be used as an antigen after linking it to a carrier such as keyhole limpet hemocyanin (KLH) or the like. Animals to be immunized are not limitative, but it is preferable to select and use an animal capable of producing the desired antibody among those conventionally used in immunological experiments by those skilled in the art such as rat, mouse, rabbit, sheep, horse, chicken, goat, pig, cattle and the like.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An antibody-producing cell such as a splenic cell, a lymphocyte and the like is taken from an immunized animal. The cell is fused with a myeloma cell strain or the like according to a known method using polyethylene glycol, Sendai virus, an electric pulse or the like to produce a hybridoma. Thereafter, acloneproducing an antibody which binds to the protein of the present invention is selected and cultured. By purifying a supernatant of the culture of the selected clone, the monoclonal antibody is obtained. The purification may be conducted by suitably combining known methods such as salting-out, ion exchange chromatography, affinity chromatography and the like.

The antibody is also obtained by a genetic engineering technique. For example, an mRNA is obtained from a splenic cell or a lymphocyte of an animal immunized with the protein of the present invention or its partial polypeptide, or from a hybridoma producing a monoclonal antibody against the protein of the present invention or its partial polypeptide. Based on the thus-obtained mRNA, a cDNA library is constructed. A cell is transformed using this cDNA library and a clone producing the antibody which reacts with the antigen is screened and the thus-screened clone is cultured. The desired antibody can be purified from the culture mixture by combined known methods. When the antibody is used for therapy, a humanized antibody is preferable in view of immunogenicity. The humanized antibody can be prepared by immunizing a mouse whose immune system has replacedwith a human immune system (see, for example, Nat. Genet., Vol. 15, pp. 146-157 (1997)). Alternatively, the humanized antibody can be engineered using a hypervariable region of the monoclonal antibody (Method in Enzymology, Vol. 203, pp. 99-121 (1991)).

<Screening method>

The present invention relates to a method of screening for an agent capable of controlling the function or the expression of the protein of the present invention, which comprises using the protein of the present invention, a transformant expressing said protein, the DNA of the present invention, a recombinant vector comprising said DNA, a transformant produced by transforming with said vector or a mt0039 gene transferred non-human mammalian animal of the present invention.

More specifically, the screening method includes:

(1) a method of evaluating an activity of the protein of the invention in the presence/absence of a candidate;
(2) a method of screening for an agent capable of controlling the expression of the protein of the present invention by comparing an expression level of the protein or the gene of the present invention in the presence/absence of a candidate; and the like.

Example of the method (1) includes

(1-1) a method of evaluating an activity of a candidate for the binding to MT0039;
(1-2) a method of identifying an agent capable of modulating a serine protease inhibitory activity of the protein of the present invention by evaluating the inhibitory activity in the presence/absence of a candidate;
(1-3) a method of identifying an agent capable of controlling an activity of enhancing the induction of apoptosis by tumor necrosis factor by evaluating the activity of a transformant expressing MT0039;

and the like. As (1-1) or (1-2), a binding assay between MT0039 and a candidate, a method of confirming the change in protease activity by adding a candidate into an assay system where serine protease and its substrate are present together with MT0039, and the like are exemplified.

When only serine protease and its substrate are present in a system, the decomposition of the substrate is observed. If MT0039 is further present in the above system, MT0039 is expected to inhibit the serine protease activity since MT0039 binds to serine protease irreversively. If the recovery in serine protease activity is observed by additionally incorporating a candidate in the system, said candidate can be judged to specifically bind to MT0039 so that the serine protease inhibitory activity of MT0039 is inhibited.

Example of the method (2) is a method comprising determining an expression amount of a reporter gene such as luciferase or the like under the condition where the gene of the present invention is transcripted or translated in the presence/absence of a candidate using an expression plasmid prepared by linking the reporter gene to the DNA containing an expression control region, a 5'-untranslated region, a region near a translational initiation site, a translation region or the like of the gene mt0039 to confirm a transcriptional promotion activity or a transcriptional inhibitory activity of the candidate. The screening method of the present invention comprises the steps of contacting a candidate with the protein of the present invention, a transformant expressing said protein, the DNA of the present invention, a recombinant vector including said DNA, a transformant produced by transformation with said recombinant vector or a mt0039 gene transferred non-human mammalian animal of the present invention; detecting a difference in an activity of the protein of the present invention or an expression level of the DNA of the present invention between a group with the addition of the candidate and a group without the addition of the candidate; and selecting the candidate showing the difference as an agent capable of controlling an activity of the protein of the present invention or an agent capable of controlling the expression of the DNA of the present invention.

An agent capable of controlling an activity of the protein of the present invention may be an agent capable of either enhancing or inhibiting the activity of the MT0039 protein. An agent capable of inhibiting the activity is preferable. An agent capable of controlling the expression of the DNA of the present invention may be an agent capable of either promoting or inhibiting the expression of the mt0039 gene. An agent capable of inhibiting the expression is preferable.

For confirming whether a candidate controls an activity of the protein of the present invention or controls the expression of the DNA of the present invention, a difference in the activity of the protein or the expression level of the DNA is determined between the addition and no addition of a candidate in a system capable of confirming the activity of the protein or a system capable of confirming the expression of the DNA. The expression level of the DNA may be determined on the basis of an expression intensity of the mt0039 gene into mRNA or the protein.

Instead of the expression level of the mt0039 gene or the MT0039 protein per se, an expression level of a reporter gene may be detected. The reporter-assay system means an assay system in which an expression amount of a reporter gene arranged downstream of an transcription control region is determined so as to screen for an agent affecting the transcription control region. Examples of the transcription control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used.

The expression control region and the 5'-untranslated region of the gene of the present invention can be obtained according to a known method (see "New Experimental Protocol in Cell Engineering", published by Shujunsha Co., Ltd. (1993)).

Having function of inhibiting (or suppressing) or enhancing (or promoting) means that a determined value of the activity of the protein or the expression level of the DNA is different between a group with the addition of a candidate and a group without the addition of a candidate. For example, the inhibition (or suppression) or the enhancement (or promotion) ratio calculated by the following equation is 10% or higher, preferably 30% or higher, more preferably 50% or higher, even preferably 70% or higher, especially preferably 90% or higher.

inhibition (or suppression) or enhancement (or promotion) ratio (%) =

[absolute value of (determined value of a group without the addition of a candidate) minus (determined value of a group with

the addition of a candidate)] / (determined value of a group

without the addition of a candidate) x 100

Either inhibition or enhancement is suitably determined depending on the kind of a system capable of confirming an activity of the protein or a system capable of confirming the expression of the DNA. For example, if a system capable of confirming an activity of the protein is a system for determining an activity of enhancing the induction of apoptosis by tumor necrosis factor as shown in Example 6, an enrichment factor can be determined. When the determined value in a group with the addition of a candidate is lower than that in a group without the addition of a candidate, the candidate can be judged to have an action of inhibiting the activity of the mt0039 protein. Of course, if values from background and/or noises is contained in a determination system, they should be subtracted.

Compounds obtained through the search using the screening method or the transgenic animal described above are expected to be effective therapeutic or preventive agents for diseases caused by hyperserection or unbalanced secretion of MT0039. Non-limiting examples of a candidate include proteins, peptides, oligonucleotides, synthetic compounds, naturally occurring compounds, fermented products, cell extracts, plant extracts, animal tissue extracts and the like. The candidate may be either new or known.

EXAMPLES

The present invention will be described in more detail by referring to the following examples which are not to be construed as limiting the scope of the invention.

Example 1 : Cloning of gene mt0039

(1) Cloning from cDNA library from human thymus

The mt0039 was cloned according to the following method: A sense primer 039-S2 (5'-GGT TTT AGA TCG TTA TAA G-3') and an antisense primer 039-A2 (5'-AAG TAG TAG ACA CTG CTC C-3') were synthesized. The PCR was performed by incubating with PLATINUM Taq DNA Polymerase (Invitrogen) using Thymus of Human MTC Panel II (Clontech) at 94°C for 5 minutes (activation of Taq DNA polymerase) followed by subjecting to 40 cycles, each cycle comprising heating at 94°C for 30 seconds, at 55°C for 30 seconds, and at 72°C for 150 seconds. Next, using 0. 5 µl of the PCR products as a template, a sense primer 039-S1 (5'-ATG GAC TCT CTT GTT ACA G-3') and an antisense primer 039-A1 (5'-TTA AGG AGA GCA GAC CCT G-3') were synthesized. The nested PCR was performed by incubating with PLATINUM Taq DNA Polymerase (Invitrogen) at 94°C for 2 minutes followed by subjecting to 30 cycles, each cycle comprising heating at 94°C for 30 seconds, at 55°C for 30 seconds, and at 72°C for 150 seconds. The resultant DNA fragment of about 1.3 kb was inserted in pGEM-T Easy Vector (Promega), thereby a plasmid pGEM-T-039 was obtained.

(2) Sequencing of cDNA clone

The plasmid pGEM-T-039 obtained by the method as described in (1) was sequenced. As the result, the plasmid contained an open reading frame comprising 1278 bases represented by SEQ ID NO:1, encoding a new protein comprising 425 amino acids represented by SEQ ID NO:2. This plasmid pGEM-T-039 was deposited in International Patent Organism Depositary (IPDO) (Tsukuba City, Ibaragi Prefecture, Japan) of National Institute of Advanced Industrial Science and Technology as FERM BP-8141 on August 7, 2001.

(3) Homology analysis of cDNA clone and its deduced protein

The amino acid sequence (MT0039) comprising 425 residues translated for the full length cDNA sequence of mt0039 is shown in SEQ ID NO:2. Next, this deduced protein sequence was searched for the homology against Genbank protein data base. For searching the homology, BLAST (Altschul SF, J. Mol. Evol., Vol. 36, pp. 290-300 (1993); Altschul SF, J. Mol. Biol., Vol. 215, pp. 403-410 (1990)) was used to search for the agreement in local sequences. As the result, this protein showed the homology of 83% and the identity of 71% with mouse putative (GenBank Accession : BAB26028) over 425 residues. This protein showed the homology of 62% and the identity of 45% with human SQUAMOUS CELL CARCINOMA ANTIGEN 2 (GenBank Accession : SCC2_HUMAN) over 425 residues. And, this protein showed the homology of 62% and the identity of 45% with human SQUAMOUS CELL CARCINOMA ANTIGEN 1 (GenBank Accession : SCC1_HUMAN)

Serpins (GenBank Accession : BAB 26028, SCC2_HUMAN, SCC1_HUMAN) which were found to be closely related as the result of the searching for the homology with the translated protein sequence were multiple aligned using the multiple alignment program Clustal W (Thompson JD., Higgins DG., Gibson TJ. , Nucleic Acids Res., Vol. 22, pp. 4673-4680 (1994)). Results are shown in Fig. 1.

Motifs are represented as consensus sequences of functional sites (for example, an active site such as an enzyme; a binding site such as a ligand or an effector; a modifying site such as a phosphorylation) identified by experiments and the like. Since especially important functional sites are often conserved even after evolution, they are used as an index characterizing a conserved sequence specifically appearing in a protein expressing certain function and also a closely related protein family. Thus, it is expected to lead an interpretation which directly relates to functions by searching for a motif rather than a homology.

In PROSITE data base (http://www.expasy.ch/prosite/) which is a motif data base, a motif is represented by a pattern of a consensus sequence. Alternatively, it is represented by a profile in which a score based on an appearance frequency or the like is expressed with a matrix for each amino acid to each position within the motif. As to the functions of the deduced protein sequence, patterns were searched for PROSITE data base. As the result, the SERPIN motif at Nos. 398 to 408 [PROSITE Accession : 00284] was found. Although the pro-phe sequence positioned at the center of this motif is especially well conserved in the serpin family, it is confirmed that this sequence is also conserved in MT0039.

Using HMMER (R. Durbin, S. Eddy, A. Krogh, G. Mitchison, Cambridge University Press (1998)) which is a program for screening for a homology using a hidden Markov model, pfam (http://www.sanger.ac.uk/Pfam/) of a protein domain database was screened. As the result, serpin domain [Pfam Accession : PF00079] was found at amino acid Nos. 3 to 425 as a significantly homologous domain.

Example 2 : Analysis of expression profile by RT-PCR

The expression profile of mt0039 in tissues was analyzed by RT-PCR. A human RNA used in this analysis was as follows: A total RNA was prepared from human coronary arterial endothelial cells (HCAEC) according to a routine method. And, leukocytes from human peripheral blood were cultured in the presence or absence of 100 μg/ml of PHA for 24 hours and then a total RNA was prepared according to a routine method. Total RNAs of human brain, kidney, pancreas, liver, thymus, spleen, heart, placenta, uterus, testes, prostate, skeletal muscle and brain were obtained from Clontech. Total RNAs of large intestine and small intestine were obtained from Biochain Institute. Next, a single-stranded cDNA was synthesized from 6 μg of each total RNA using oligoDT primer according to a routine method. As a reverse transcriptase, SuperScript II RNaseH$^-$ Reverse Transcriptase (Invitrogen) was used. As primers specific for mt0039, a sense primer (5'-TAT GGT ACG CTT GGG TGC TA-3') and an antisense primer (5'-CCA GTC CGC TCT CAT TGT TT-3') were used in the PCR. The analysis demonstrated that mt0039 showed strong expression in thymus, uterus and testes and weak expression in kidney, placenta, prostate and brain (Fig. 2).

Example 3 : Expression of protein by in vitro translation

The expression of MT0039 is performed by in vitro translation using RTS500 System (Rosch Diagnostics) . In order to facilitate the purification after the expression, a construct having a His tag fused to N-terminal of MT0039 is prepared. A vector attached to RTS500 System is used as an expression vector. The construction of the expression plasmid and the synthesis of the protein are performed according to the provider' s protocol. The thus-synthesized product is purified using His Trap Kit (Amersham Pharmacia Biotech) according to Strategy Guide in Bio Basic Experiments, 2000-2001 (Amersham Pharmacia Biotech).

Example 4 : Determination of serine protease inhibitory activity

According to a routine method, 1 to 1000 nM of serine protease, 1 to 1000 μM of a synthetic substrate having para-nitroaniline (pNA) at its terminal corresponding to serine protease and 0 to 100 μM of MT0039 are added in a phosphate buffer containing 0.01% of bovine serum albumin (Sigma) and they are reacted. An amount of pNA released by the action of protease is measured by determining an absorbance at 405 nm in order to confirm whether or not MT0039 has a serine protease inhibitory activity.

Example 5 : Construction of MT0039 expression plasmid

In order to express MT0039 in a mammalian cell, a MT0039 expression plasmid (hereinafter called "pRM0039") was constructed. Briefly, a region containing ORF of MT0039 was amplified by PCR reaction with Pyrobest DNA polymerase (TAKARA BIO INC.) using pGEM-T-039 as a template. The resultant fragment was ligated to a cloning site of an expression vector from mammalian cell. The ligation product was transformed into E.coli JM109 competent cell (TAKARA BIO INC.), thereby the objective plasmid was obtained.

Example 6 : Action on apoptosis induced by tumor necrosis factor

HeLa cells (cells from human cervical carcinoma; obtained fromATCC) were incubated in Dulbecco's modified Eagle's medium containing 10% of fetal bovine serum (FBS; JRH Bioscience) at 5% $CO_2$ and 37°C for one day (BNR-110M; Espec Corp.) . Then, the medium was replaced with DMEM containing 1% of FBS, to which pRM0039, the MT0039 expression vector, was transfected using FuGENE6 (Rosche Diagnostics) and incubated for additional two days, thereby MT0039 was expressed in the cells.

The expressed cells were again suspended in DMEM containing 10% of FBS and incubated at 5% $CO_2$ and 37°C for one day, to which a human tumor necrosis factor (TNF)-α (MHR-24; MOCHIDA PHARMACEUTICAL CO., LTD.) at a final concentration of 0 to 100 ng/ml and 0 or 10 μg/ml of cycloheximide (CHX; Sigma) were added and cultured for additional four hours. The cells were harvested and detected for a histone/DNA fragment complex using an apoptosis detection ELISA kit$^{plus}$ (Rosche Diagnostics). The cells were lysated in the provider's lytic buffer and the lysate was transferred into a microplate coated with streptavidin. Then, an anti-histone antibody labeled with biotin and an anti-DNA antibody labeled with peroxidase were added and they were reacted at room temperature for two hours. The wells were washed with an incubation buffer and then colored by adding 2,2'-azino-di[3-ethylbenzthiazoline sulfonic acid] (ABTS). The detection was performed by determining an absorbance at measurement wavelength of 405 nm/ reference wavelength of 490 nm using a microplate reader (iEMS Reader; Labsystems). The degree of apoptosis was evaluated using Enrichment Factor of nucleosome as an indicator. This was obtained according to the manual attached to the kit by subtracting an absorbance of unreacted ABTS as a blank from an absorbance of each sample and then calculating the ratio of an average of the absorbances of the samples to an average of an absorbance of cells reacted with neither THF-α nor CHX, the latter average being 1. As illustrated in Fig. 3, MT0039 enhanced the induction of apoptosis by TNF-α in HeLa cells. The enhancement by MT0039 in apoptosis induced by TNF-α as compared with that of negative controls (a group of cells not subjected to any transfection and a control, i.e. cells transfected with an expression plasmid to which the mt0039 gene was not integrated) was at least twice over the TNF concentration of 0.1 to 100 ng/ml. At the TNF concentration of 100 ng/ml, it is considered that a saturation may occur due to excessive TNF. Over the TNF concentration of 0.1 to 10 ng/ml which is believed to be suitable, the enhancement by MT0039 was at least three times that of the negative controls.

Example 7 : Purification of MT0039 protein

The recombinant MT0039 protein was isolated and purified by metal chelate affinity chromatography using His-Trap kit (Amersham Pharmacia Biotech). COS-1 cells (cells from monkey kidney; obtained by ATCC) were cultured in Dulbecco's modified Eagle's medium (DMEM; Sigma) containing 10% of fetal bovine serum (FBS; JRH Bioscience) at 5% $CO_2$ and 37°C for one day (BNR-110M; Espec Inc.). The medium was replaced with DMEM containing 1% of FBS, to which pRM0039, the MT0039 expression vector, was transfected using FuGENE 6 (Rosche Diagnostics) and cultured for additional two days, thereby MT0039 was expressed in the cells. The cells were harvested in a 50 ml centrifuge tube and the thus-harvested pellets were suspended in a total volume of 5 ml of an ice-cold Start buffer [Sigma; 1 x phosphate buffer (D8537), pH 7.4, 10 mM imidazole, protease inhibitor cocktail (SIGMA: 1 ml of cocktail solution per 20 g of wet weight)] and homogenized by sonification. Cell debris were precipitated by centrifuging (TOMY MRX-150; 10000 rpm) at 4°C for 30 minutes and then the supernatant was collected and filtered through a 0.45 μm filter (Millipore). The filtrate was applied to a $Ni^2$-charged agarose column equilibrated with 10ml of Start buffer [1 x phosphate buffer, pH 7.4, 10 mM imidazole] and eluted with 5 ml of an elution buffer [1 x phosphate buffer, pH 7.4, 100 mM/300 mM/500 mM imidazole].

A fraction containing the eluted MT0039 was collected, ultrafiltered through Centricon 10 (Millipore) and concentrated. The concentrate was dialyzed against a dialyzing outer solution (20 mM Tris-Cl, pH 7.4, 300 mM NaCl) using a dialysis membrane of MW 14000 cut. The MT0039 protein-containing fraction was subjected to the electrophoresis on 10% SDS-polyacrylamide gel and then silver stained (2D-silver staining reagent "Dai-ichi"; Daiichi Pure Chemicals Co., Ltd.). The results are illustrated in Fig. 4. A single band was observed at about 55000 Da. Although MT0039 has the theoretical MW of about 48000 Da since it comprises 425 a.a., the determined MW of MT0039 was higher than the theoretical MW. It is assumed that the difference in MW will be brought about by the addition of a sugar chain due to the presence of a sugar chain binding site on the sequence of MT0039 and thereby the band is shifted.

Example 8 : Protease inhibitory profiles of MT0039

The following experiments were performed using the MT0039 protein purified in Example 7.

(8-1) Detection of trypsin-type serine protease inhibitory action

(8-1-1) Detection of trypsin inhibitory activity

15 ng/ml of trypsin (Athens Research and Technology) , 0.4 mmol/L of a synthetic substrate (S-2222; Chromogenix) and 10 μg/ml of MT0039 were added in a Tris-buffered saline containing 0.1 w/v% of bovine serum albumin (BSA; Sigma) and they were reacted at 37°C for 20 minutes. An amount of para-nitroaniline (pNA) released by the action of trypsin was measured by determining an absorbance at 405 nm (iEMS Reader; Labsystems). Simultaneously, an amount of pNA released by the action of trypsin in a MT0039-free system was measured. From these values, the trypsin inhibitory activity of MT0039 was calculated. 10 μg/ml of MT0039 inhibited 97% of the activity of trypsin at 15 ng/ml.

(8-1-2) Detection of thrombin inhibitory activity

0.1 U/ml of thrombin (Sigma), 0.4 mmol/L of a synthetic substrate (S-2238; Chromogenix) and 10 μg/ml of MT0039 were added in a Tris-buffered saline containing 0.1 w/v% of BSA and they were reacted at 37°C for 20 minutes. An amount of pNA released by the action of thrombin was measured by determining an absorbance at 405 nm. Simultaneously, an amount of pNA released by the action of thrombin in a MT0039-free system was measured. From these values, the thrombin inhibitory activity of MT0039 was calculated. 10 μg/

ml of MT0039 showed no inhibitory activity on thrombin at 0.1 U/ml.

(8-1-3) Detection of kallikrein inhibitory activity

2 mU/ml of kallikrein (Sigma), 0.4 mmol/L of a synthetic substrate (S-2302; Chromogenix) and 10 μg/ml of MT0039 were added in a Tris-buffered saline containing 0.1 w/v% of BSA and they were reacted at 37°C for 30 minutes. An amount of pNA released by the action of kallikrein was measured by determining an absorbance at 405 nm. Simultaneously, an amount of pNA released by the action of kallikrein in a MT0039-free system was measured. From these values, the kallikrein inhibitory activity of MT0039 was calculated. 10 μg/ml of MT0039 showed no inhibitory action on kallikrein at 2 mU/ml.

(8-1-4) Detection of activated protein C (APC) inhibitory activity

100 ng/ml of APC (American Diagnostica), 0.4 mmol/L of a synthetic substrate (S-2362; Chromogenix) and 10 μg/ml of MT0039 were added in a Tris-buffered saline containing 0.1 w/v% of BSA and they were reacted at 37°C for 60 minutes. An amount of pNA released by the action of APC was measured by determining an absorbance at 405 nm. Simultaneously, an amount of pNA released by the action of APC in a MT0039-free system was measured. From these values, the APC inhibitory activity of MT0039 was calculated. 10 μg/ml of MT0039 showed no inhibitory action on APC at 100 ng/ml.

(8-1-5) Detection of plasmin inhibitory activity

10 mU/ml of plasmin (Sigma), 0.4 mmol/L of a synthetic substrate (S-2251; Chromogenix) and 10 μg/ml of MT0039 were added in a Tris-buffered saline containing 0.1 w/v% of BSA and they were reacted at 37°C for 20 minutes. An amount of pNA released by the action of plasmin was measured by determining an absorbance at 405 nm. Simultaneously, an amount of pNA released by the action of plasmin in a MT0039-free system was measured. From these values, the plasmin inhibitory activity of MT0039 was calculated. 10 μg/ml of MT0039 inhibited 87% of the activity of plasmin at 10 mU/ml.

(8-1-6) Detection of tissue plasminogen activator (t-PA) inhibitory activity

1000 IU/ml of t-PA (Activacin; Kyowa Hakko Kogyo Co., Ltd.), 0.8 mmol/L of a synthetic substrate (S-2288; Chromogenix) and 10 μg/ml of MT0039 were added in a Tris-buffered saline containing 0.1 w/v% of BSA and they were reacted at 37°C for 30 minutes. An amount of pNA released by the action of t-PA was measured by determining an absorbance at 405 nm. Simultaneously, an amount of pNA released by the action of t-PA in a MT0039-free system was measured. From these values, the t-PA inhibitory activity of MT0039 was calculated. 10 μg/ml of MT0039 showed no inhibitory action on t-PA at 1000 IU/ml.

(8-1-7) Detection of activated X factor (FXa) inhibitory activity

0.02 U/ml of FXa (Enzyme Research), 0.4 mmol/L of a synthetic substrate (S-2222) and 10 μg/ml of MT0039 were added in a Tris-buffered saline containing 0.1 w/v% of BSA and they were reacted at 37°C for 30 minutes. An amount of pNA released by the action of FXa was measured by determining an absorbance at 405 nm. Simultaneously, an amount of pNA released by the action of FXa in a MT0039-free system was measured. From these values, the FXa inhibitory activity of MT0039 was calculated. 10 μg/ml of MT0039 hardly inhibited the activity of FXa at 0.02 U/ml.

(8-1-8) Detection of urokinase-type plasminogen activator (u-PA) inhibitory activity

600 U/ml of u-PA (Kowa Company Ltd.), 0.4 mmol/L of a synthetic substrate (S-2222) and 10 μg/ml of MT0039 were added in a Tris-buffered saline containing 0.1 w/v% of BSA and they were reacted at 37°C for 20 minutes. An amount of pNA released by the action of u-PA was measured by determining an absorbance at 405 nm. Simultaneously, an amount of pNA released by the action of u-PA in a MT0039-free system was measured. From these values, the u-PA inhibitory activity of MT0039 was calculated. 10 μg/ml of MT0039 showed no inhibitory action on u-PA at 600 U/ml.

(8-1-9) Detection of granzyme B inhibitory activity

20 μg/ml of granzyme (Calbiochem), 1 mmol/L of a synthetic substrate (Boc-Ala-Ala-Asp-pNa; Bachem) and 100 μg/ml of MT0039 were added in a phosphate-buffered saline (Sigma) containing 4 mmol/L of dithiothreitol (Sigma) and they were reacted at 37°C for 30 minutes. An amount of pNA released by the action of granzyme B was measured by determining an absorbance at 405 nm. Simultaneously, an amount of pNA released by the action of granzyme B in a MT0039-free system was measured. From these values, the granzyme B inhibitory activity of MT0039 was calculated. 100 μg/ml of MT0039 hardly inhibited the activity of granzyme B at 10 μg/ml.

(8-2) Detection of chymotrypsin and elastase-type serine protease inhibitory action

(8-2-1) Detection of chymotrypsin inhibitory activity

100 ng/ml of chymotrypsin (Athens Research and Technology), 0.4 mmol/L of a synthetic substrate (S-2586; Chromogenix) and 10 μg/ml of MT0039 were added in a Tris-buffered saline containing 0.1 w/v% of BSA and they were reacted at 37°C for 20 minutes. An amount of pNA released by the action of chymotrypsin was measured by determining an absorbance at 405nm. Simultaneously, an amount of pNA released by the action of chymotrypsin in a MT0039-free system was measured. From these values, the chymotrypsin inhibitory activity of MT0039 was calculated. 10 μg/ml of MT0039 showed no inhibitory action on chymotrypsin at 100 ng/ml.

(8-2-2) Detection of neutrophil elastase inhibitory activity

2 μg/ml of neutrophil elastase (Athens Research and Technology), 0.2 mmol/L of a synthetic substrate (S-2484; Chromogenix) and 20 μg/ml of MT0039 were added in a Tris-buffered saline containing 0.1 w/v% of BSA and they were reacted at 37°C for 5 minutes. An amount of pNA released by the action of neutrophil elastase was measured by determining an absorbance at 405 nm. Simultaneously, an amount of pNA released by the action of neutrophil elastase in a MT0039-free system was measured. From these values, the neutrophil elastase inhibitory activity of MT0039 was calculated. 20 μg/ml of MT0039 showed no inhibitory action on neutrophil elastase at 2 μg/ml.

(8-2-3) Detection of cathepsin G inhibitory activity

1 μg/ml of cathepsin G (Athens Research and Technology), 0.4 mmol/L of a synthetic substrate (N-Suc-Ala-Ala-Pro-Phe-pNA; Sigma) and 100 μg/ml of MT0039 were added in a Tris-buffered saline containing 0.1 w/v% of BSA and they were reacted at 37°C for 60 minutes. An amount of pNA released by the action of cathepsin G was measured by determining an absorbance at 405 nm. Simultaneously, an amount of pNA released by the action of cathepsin G in a MT0039-free system was measured. From these values, the cathepsin G inhibitory activity of MT0039 was calculated. 100 μg/ml of MT0039 inhibited 76% of the activity of cathepsin G at 1 μg/ml.

(8-3) Detection of cysteine protease inhibitory action

(8-3-1) Detection of cathepsin K inhibitory activity

25 ng/ml of cathepsin K (Bioscience laboratory of MOCHIDA PHARMACEUTICAL CO., LTD.), 10 μmol/L of a synthetic substrate (Z-Leu-Arg-AMC; Enzyme System Products) and 1 μg/ml of MT0039 were added in an acetate buffer and they were reacted at 37°C for 10 minutes. An amount of 7-amino-4-methyl coumarin (AMC) released by the action of cathepsin K was measured by determining a fluorescence (excitation wavelength= 355 nm and determination wavelength= 460 nm). Simultaneously, an amount of AMC released by the action of cathepsin K in aMT0039-free system was measured. From these values, the cathepsin K inhibitory activity of MT0039 was calculated. 1 μg/ml of MT0039 hardly inhibited the activity of cathepsin K at 25 ng/ml.

(8-3-2) Detection of cathepsin L inhibitory activity

50 ng/ml of cathepsin L (CALBIOCHEM), 10 μmol/L of a synthetic substrate (Z-Leu-Arg-AMC) and 1 μg/ml of MT0039 were added in an acetate buffer and they were reacted at 37°C for 10 minutes. An amount of AMC released by the action of cathepsin L was measured by determining a fluorescence (excitation wavelength= 355 nm and determination wavelength= 460 nm). Simultaneously, an amount of AMC released by the action of cathepsin K in a MT0039-free system was measured. From these values, the cathepsin L inhibitory activity of MT0039 was calculated. 1 μg/ml of MT0039 showed no inhibitory activity on cathepsin L at 50 ng/ml.

## EFFECT OF THE INVENTION

MT0039 of the present invention can be utilized in medicines as a protease inhibitory protein as such. In addition, it is useful for designing its mimic low molecules and identifying agents capable of inhibiting the binding to protease. And, it is useful for developing drugs capable of affecting new control mechanisms in vivo.

BRIEF DESCRIPTION OF DRAWINGS

Fig. 1 shows the amino acid sequence alignment between MT0039 and other known proteins.

Fig. 2 shows the expression profile of the mt0039 gene in human organs and various cells.

Fig. 3 shows the results of determining the activity of MT0039 in enhancing the induction of apoptosis by TNF-α as described in Example 6. In this figure, the white square represents the determined value of a cell not subjected to any transfection (non-transfection group). The black square represents the determined value of a cell transfected with the expression plasmid in which the mt0039 gene was not incorporated (control). The black triangle represents the determined value of a cell transfected with the MT0039 expressing plasmid (MT0039) .

Fig. 4 shows the silver staining of the recombinant MT0039 protein. The left lane illustrates a molecular weight marker and the right lane illustrates the MT0039 containing fraction after the electrophoresis.

## Claims

1. The following protein (a) or (b):

   (a) a protein comprising the amino acid sequence represented by SEQ ID NO:2; or
   (b) a serine protease inhibitory protein comprising an amino acid sequence wherein deletion, substitution or addition of one or more amino acids has occurred in the amino acid sequence of SEQ ID NO:2 and having an activity of enhancing the induction of apoptosis by tumor necrosis factor.

2. A DNA encoding the protein as defined in claim 1.

3. The following DNA (a) or (b):

   (a) a DNA comprising the nucleotide sequence represented by SEQ ID NO:1; or
   (b) a DNA having at least 90% identity with the DNA of SEQ ID NO:1 and encoding a serine protease inhibitory protein having an activity of enhancing the induction of apoptosis by tumor necrosis factor.

**4.** A recombinant vector including the DNA as defined in claim 2 or 3.

**5.** A transformant produced by transformation with the recombinant vector as defined in claim 4.

**6.** An antisense nucleic acid inhibiting the expression of the protein as defined in claim 1.

**7.** An antisense nucleic acid as defined in claim 6 wherein the nucleic acid sequence is a sequence complementary to the entire or a part of the DNA as defined in claim 2.

**8.** An antibody against the protein as defined in claim 1.

**9.** A method of screening for an agent capable of controlling an activity of the protein as defined in claim 1, which comprises contacting the protein or the transformant expressing the protein with a candidate.

**10.** A method of screening for an agent capable of controlling the expression of the DNA as defined in claim 2 or 3, which comprises contacting the recombinant vector as defined in claim 4 or the transformant as defined in claim 5 with a candidate.

Fig. 1

```
SCC1_HUMAN    MNSLSEANTKFMFDLFQQFRKS-KENNIFYSPISITSALGMVLLGAKDNTAQQIKKVLHF
SCC2_HUMAN    MNSLSEANTKFMFDLFQQFRKS-KENNIFYSPISITSALGMVLLGAKDNTAQQISKVLHF
MT0039        MDSLVTANTKFCFDLFQEIGKDDRHKNIFFSPLSLSAALGMVRLGARSDSAHQIDEVLHF
BAB26028      MDSLTAANNKFCFDFFREISKDDAHKNIFVCPLSLSAAFGMVRLGARGDSAHQIDEALHF
              *:**  **.** **:*::: *.  .:*** .*:*:::*:*** ***:.::*:**.:.***

SCC1_HUMAN    DQVTENTTGKA---------------------ATYHVDRS----------GNYHHQFQKL
SCC2_HUMAN    DQVTENTTEKA---------------------ATYHVDRS----------GNYHHQFQKL
MT0039        NEFSQNESKEP-DPCLKSNKQKVLADSSLEGQKKTTEPLDQQAGSLNNESGLYSCYFGQL
BAB26028      NELSKDEHKEPNDPSPQSESK--ASDSSLEGQKQTSASQDQQ-GESTNDHQLLGCHFGKL
              ::.::: :.                       ::  *:.          :    * :*

SCC1_HUMAN    LTEFNKSTDAYELKIANKLFGEKTYLFLQEYLDAIKKFYQTSVESVDFANAPEESRKKIN
SCC2_HUMAN    LTEFNKSTDAYELKIANKLFGEKTYQFLQEYLDAIKKFYQTSVESTDFANAPEESRKKIN
MT0039        LSKLDRIKTDYTLSIANRLYGEQEFPICQEYLDGVIQFYHTTIESVDFQKNPEKSRQEVN
BAB26028      LSRIDRDKSYYTLSMANRLYGEQEFPICSEYSDDVTEFFHTTVESVDFQKDSEKSRQEIN
              *:.::: .  * *.:**:*:**: : : .** * : :*::*::**.** : .*:**:::*

SCC1_HUMAN    SWVESQTNEKIKNLIPEGNIGSNTTLVLVNAIYFKGQWEKKFNKEDTKEEKFWPNKNTYK
SCC2_HUMAN    SWVESQTNEKIKNLFPDGTIGNDTTLVLVNAIYFKGQWENKFKKENTKEEKFWPNKNTYK
MT0039        FWVECQSQGKIKELFSKDAINAETVLVLVNAVYFKAKWETYFDHEDTVDAPFCLNANENK
BAB26028      FWVESQSQGKIKELFGKEAIDNSTVLVLVNAVYFKAKWEREFNSENTVDASFCLNENEKK
               ***.*:: ***:*: .  *. .*.******:***.:**  *. *:* :  *  * *  *

SCC1_HUMAN    SIQMMRQYTSFHFASLEDVQAKVLEIPYKGKDLSMIVLLPNE----IDGLQKLEEKLTAE
SCC2_HUMAN    SVQMMRQYNSFNFALLEDVQAKVLEIPYKGKDLSMIVLLPNE----IDGLQKLEEKLTAE
MT0039        SVKMMTQKGLYRIGFIEEVKAQILEMRYTKGKLSMFVLLPSHSKDNLKGLEELERKITYE
BAB26028      TVKMMNQKGKFRIGFIDELQAQILEMKYAMGKLSMLVLLPSCSEDNVNSLQELEKKINHE
              :::** *   :.:. .::::*::**:. *   .***:****.      :..*::**.*:. *

SCC1_HUMAN    KLNEWTSLQNMRETRVDLHLPRFKVEESYDLKDTLRTMGMVDIFNG-DADLSGMTGSRGL
SCC2_HUMAN    KLNEWTSLQNMRETCVDLHLPRFKMEESYDLKDTLRTMGMVNIFNG-DADLSGMTWSHGL
MT0039        KMVAWSSSENMSEESVVLSFPRFTLEDSYDLNSILQDMGITDIFDETRADLTGISPSPNL
BAB26028      KLLAWSSSENLSEKPVAISFPQFNLEDSYDLKSILQDMGIKDVFDETKADLTGISKSPNL
              *:: *:* :*: *  * : :*:*.:*:****:. *: **: ::*:   ***:*:: * .*

SCC1_HUMAN    VLSGVLHKAFVEVTEEGAEAAAATAVVGFGSSPASTNEEFHCNHPFLFFIRQNKTNSILF
SCC2_HUMAN    SVSKVLHKAFVEVTEEGVEAAAATAVVVVELSSPSTNEEFCCNHPFLFFIRQNKTNSILF
MT0039        YLSKIIHKTFVEVDENGTQAAAATGAVVSERS-LRSWVEFNANHPFLFFIRHNKTQTILF
BAB26028      YLSKIVHKTFVEVDEMGTQAAAASGVVAAEKA-LPSWVEFNANHPFLFFIRHNPTQSLLF
               :* ::**:**** * *.:****:..*      :   :  ** .*********:* *:::**

SCC1_HUMAN    YGRFSSP
SCC2_HUMAN    YGRFSSP
MT0039        YGRVCSP
BAB26028      CGRVYCP
               **. .*
```

Fig. 2

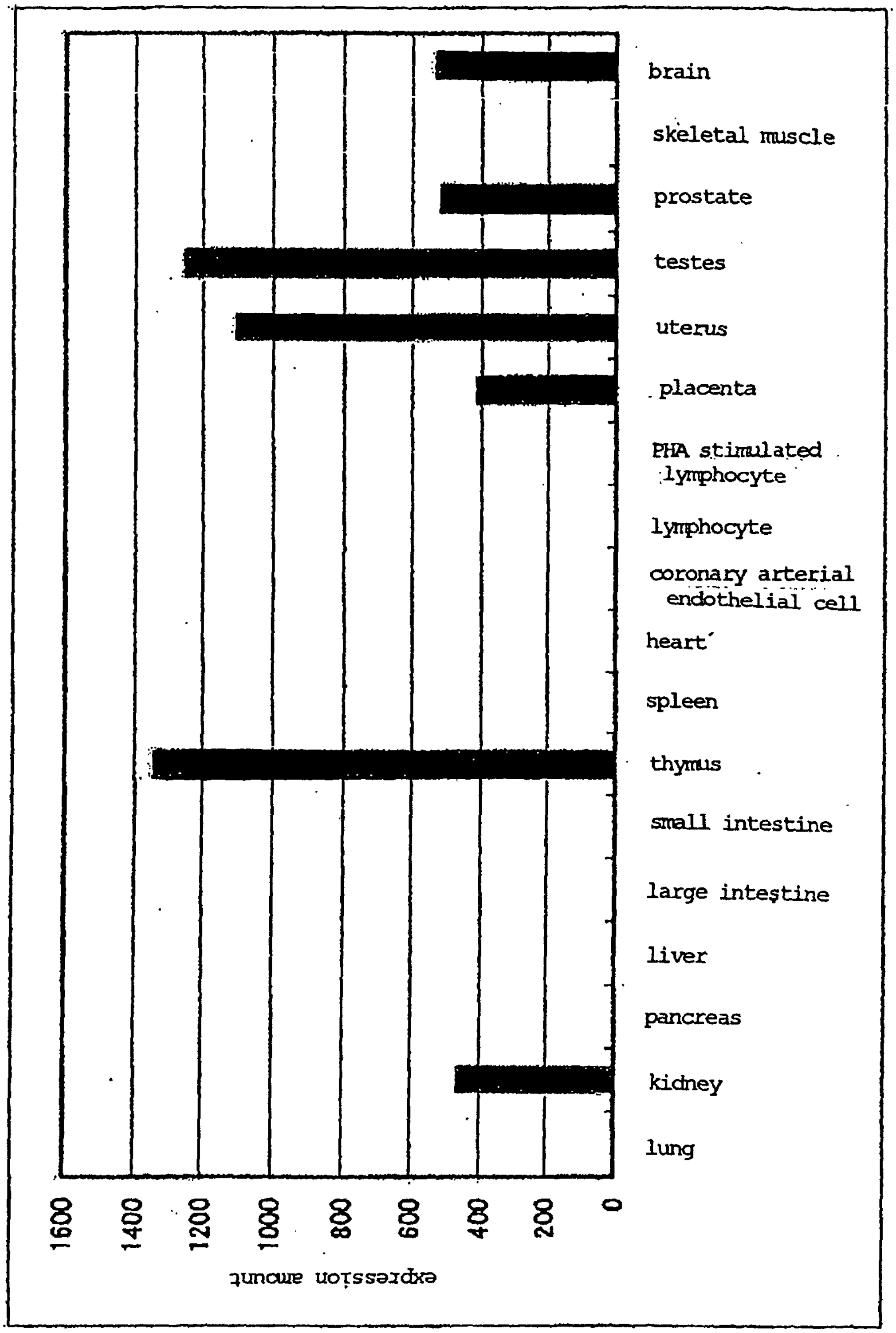
brain
skeletal muscle
prostate
testes
uterus
placenta
PHA stimulated lymphocyte
lymphocyte
coronary arterial endothelial cell
heart
spleen
thymus
small intestine
large intestine
liver
pancreas
kidney
lung
1600
1400
1200
1000
800
600
400
200
0
expression amount

Fig. 3

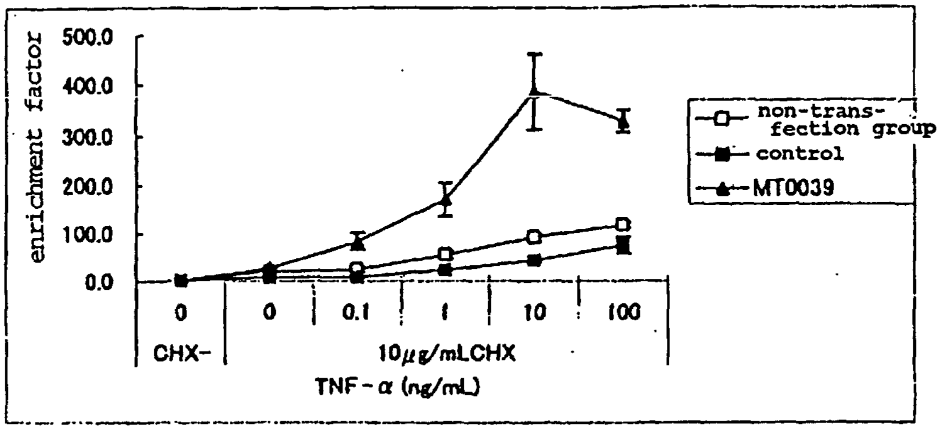
enrichment factor
500.0
400.0
300.0
200.0
100.0
0.0
0
0
0.1
1
10
100
CHX-
10μg/mLCHX
TNF-α (ng/mL)
non-transfection group
control
MT0039

Fig. 4

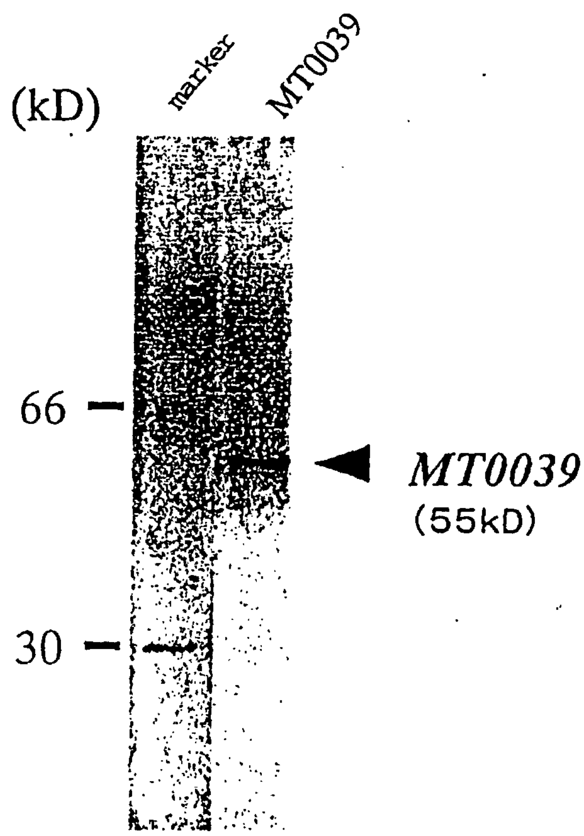
(kD)
marker
MT0039
66
30
MT0039
(55kD)

## INTERNATIONAL SEARCH REPORT

International application No.
PCT/JP02/08948

**A. CLASSIFICATION OF SUBJECT MATTER**
Int.Cl[7] C12N15/12, 9/99, 5/10, C12Q1/68, 1/02, C07K16/38

According to International Patent Classification (IPC) or to both national classification and IPC

**B. FIELDS SEARCHED**

Minimum documentation searched (classification system followed by classification symbols)
Int.Cl[7] C12N15/00-15/90, 9/00-9/99, C07K14/00-14/825

Documentation searched other than minimum documentation to the extent that such documents are included in the fields searched

Electronic data base consulted during the international search (name of data base and, where practicable, search terms used)
MEDLINE(STN), GenBank/EMBL/DDBJ/GeneSeq, SwissProt/PIR/GeneSeq, BIOSIS/WPI(DIALOG), JICST FILE(JOIS)

**C. DOCUMENTS CONSIDERED TO BE RELEVANT**

| Category* | Citation of document, with indication, where appropriate, of the relevant passages | Relevant to claim No. |
|---|---|---|
| P,A | ASKEW Y.S. et al., SERPINB12 is a Novel Member of the Human ov-serpin Family That is Widely Expressed and Inhibits Trypsin-like Serine Proteinases. J.Biol.Chem. Dec.2001, Vol.276, No.52, pages 49320 to 49330 | 1-10 |
| A | BUISSON A. et al., Up-regulation of a serine protease inhibitor in astrocytes mediates the neuro-protective activity of transforming growth factor β1. FASEB J. 1998, Vol.12, No.15, pages 1683 to 1691 | 1-10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 September, 2002 (25.09.02) | 08 October, 2002 (08.10.02) |
| Name and mailing address of the ISA/ Japanese Patent Office | Authorized officer |
| Facsimile No. | Telephone No. |

Form PCT/ISA/210 (second sheet) (July 1998)